# EUROPEAN PATENT APPLICATION

(11) **EP 2 620 141 A1**
(43) Date of publication of application: **31.07.2013**
(21) Application number: 12000467.6
(22) Date of filing: 25.01.2012
(51) Int. Cl.: A61K 9/70, A61K 31/122

(54) **Thin film drug delivery system for the transmucosal administration of a 1,4-benzoquinone derivative**

(71) Applicant: Santhera Pharmaceuticals (Schweiz) AG, 4410 Liestal (CH)
(72) Inventor: The designation of the inventor has not yet been filed
(74) Representative: Grünecker, Kinkeldey, Stockmair & Schwanhäusser

(57) **Abstract**

The present invention is directed to a thin film drug delivery system for the transmucosal administration of a pharmaceutical drug comprising 0.01-80% by weight of an active ingredient of a 1,4-benzoquinone derivative of formula (I) and 20-99.99% by weight of a carrier material.

## Description

The present invention relates to a thin film drug delivery system for transmucosal administration of 1,4-benzoquinones. Specifically, the present invention relates to a transmucosal administration system to administer 2-(10-Hydroxydecyl)-5,6-dimethoxy-3-methyl-cyclohexa-2,5-diene-1,4-dione (idebenone) and its analogues via a thin film formulation.

Idebenone is a short-chain benzoquinone used in a variety of medical applications. Similar to coenzyme Q10, idebenone undergoes reduction/oxidation cycles in living organisms and reduced idebenone is an antioxidant and radical scavenger (A. Mordente, G. E. Martorana, G. Minotti, B. Giardina, Chem. Res. Toxicol. 11 (1998), 54-63). It is known that idebenone protects cell membranes and mitochondria from oxidative damage because of its ability to inhibit lipid peroxidation (M. Suno, M. Shibota, A. Nagaoka, Arch. Gerontol. Geriatr. 8 (1989), 307-311). In addition, idebenone interacts with the ETC, preserving ATP formation in ischemic states (Y. Sugiyama, et al. J Pharmacobio Dyn 8 (1985), 1006-1017; Y. Sugiyama and Fujita T, FEBS Lett. 184 (1985) 48-51). It has been shown that the compound stimulates nerve growth factor, a characteristic that could be important for the treatment of Alzheimer's and other neurodegenerative diseases (K. Yamada, A. Nitta, T. Hasegawa, K. Fuji, M. Hiramatsu, T. Kameyama, Y. Furukawa, K. Hayashi, T. Nabeshima, Behav. Brain Res. 83 (1997), 117-122). The compound has also been suggested for the treatment of Friedreich's ataxia and other mitochondrial and neuromuscular diseases (A. O. Hausse, Y. Aggoun, D. Bonnet, D. Sidi, A. Munnich, A. Rotig, P. Rustin, Heart 87 (2002), 346-349; Di Prospero N.A., Baker A., Jeffries N, Fischbeck K.H. Lancet Neurol 6 (2007) 878-886).

As a lipophilic compound, idebenone is well absorbed in the gastrointestinal tract after conventional oral administration, which is the normal route for administering said compound, and has a high food effect. Dosage forms such as film-coated tablets or sugar-coated tablets have been used in clinical trials and as marketed product. In the course of our investigations on the pharmacological profile of idebenone, we discovered that the compound, after being absorbed in the gut, is metabolized very quickly during its first passage through the liver ("first-pass-effect"). Experiments showed that idebenone is extensively metabolized during its first passage through the liver. Hepatic metabolism of idebenone results in side chain oxidation, reduction of the quinone ring, sulphate and glucuronide conjugation and subsequent renal excretion (K Kutz, et al. J Neurol (2009) 256 Suppl. 1, 31-35). The high liver metabolism greatly reduces the potentially high plasma levels of the pharmacologically active idebenone. Because of this strong first pass metabolism, oral administration of idebenone requires high doses of the compound to achieve pharmacologically efficacious plasma levels in the body. Said high doses can result in unwanted side effects such as diarrhea.

In addition, the requisite swallowing of oral formulations of idebenone inflicts difficulties in the practical administration to patients with swallowing problems, e.g. a patient with a serious neuromuscular disease such as Duchenne muscular dystrophy or Friedreich's ataxia, elderly or pediatric patients.

The problem underlying the present invention is to provide a delivery system for 1,4-benzoquinones which avoids the first-pass metabolism observed after conventional oral administration and gastrointestinal absorption, and which facilitates the administration of 1,4-benzoquinones to patients with swallowing problems.

### Summary of the invention

Said problem has been solved by a thin film drug delivery system for transmucosal administration according to claim 1.

The thin film releases the active ingredient directly to the mucosa or partly into the saliva in the oral cavity when attached to the oral mucosa. The active ingredient is absorbed through the mucosa primarily in the oral cavity, thus avoiding the first-pass metabolism observed after conventional oral administration and gastrointestinal absorption. This dosage form is also designated as oral wafer.

The active ingredient is a 1,4-benzoquinone, preferably idebenone, having a reversibly reducible quinone ring, with a lipophilic side chain.

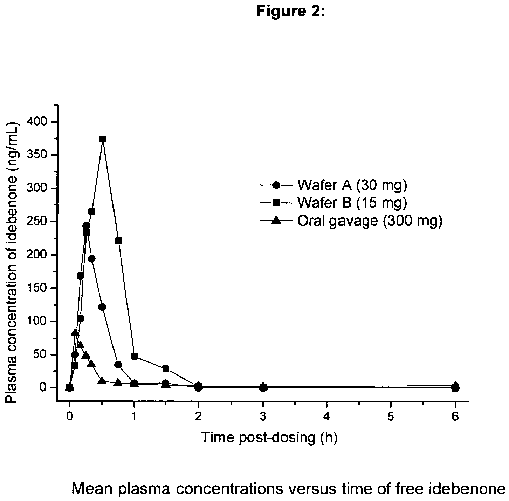

Surprisingly, it has been shown (see Figure 2) that the plasma levels of idebenone after oromucosal administration of wafer A (containing 30 mg) and wafer B (containing 15 mg as a solid solution) are significantly higher compared to oral administration (300 mg/kg as microemulsion) in the same Beagle dogs (n=3).

### Detailed description of the invention

The present invention relates to a thin film drug delivery system for the transmucosal administration of a pharmaceutical drug comprising 0.01-80% by weight of an active ingredient of the formula (I) 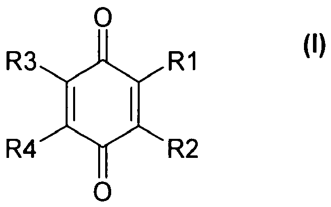 wherein
R1 is a C1-4 alkyl group;
R2 is a hydrogen atom, an optionally substituted alkyl or an optionally substituted alkenyl group;
each of R3 and R4 represents independently an optionally substituted C1-6 alkyl or C1-3 alkoxy group, or
R3 and R4 taken together represent an optionally substituted butadienylene group; and
20-99.99% by weight of a carrier material.

In a preferred embodiment in combination with any of the above or below embodiments, R1 is a C1-4 alkyl group, i.e. an alkyl group containing 1, 2, 3 or 4 carbon atoms, more preferably selected from the group consisting of methyl, ethyl, propyl and butyl, in particular, methyl. In another preferred embodiment in combination with any of the above or below embodiments, R1 is a C1-3 alkyl group.

In a preferred embodiment in combination with any of the above or below embodiments, R2 is hydrogen, a C1-50 alkyl, preferably C1-22 alkyl, or a C2-50 alkenyl, preferably C2-22 alkenyl, wherein the alkyl or alkenyl may be substituted by 1 to 10 substituents selected from the group consisting of (i) C1-4 alkyl, (ii) hydroxy, (iii) oxo, (iv) amino, (v) mono-C1-6 alkylamino, (vi) di-C1-6 alkylamino, (vii) carboxy, (viii) C1-4 alkoxycarbonyl, (ix) C6-14 aryl, which may be substituted by 1 or 2 substituents selected from the group consisting of C1-4 alkyl, hydroxy, carboxy and C1-6 alkoxycarbonyl, (x) 5- or 6-membered heterocyclic group, which may be substituted by 1 or 2 substituents selected from the group consisting of C1-4 alkyl, hydroxy, carboxy and C1-6 alkoxycarbonyl, and (xi) halogen; and wherein R2 is preferably a C6-14 alkyl optionally substituted by hydroxy.

In a preferred embodiment in combination with any of the above or below embodiments, R2 is an optionally substituted C1-50 alkyl group, preferably an optionally substituted C1-22 alkyl, i.e. an alkyl group containing 1, 2, 3, 4, 5, 6, 7, 8, 9, 10, 11, 12, 13, 14, 15, 16, 17, 18, 19, 20, 21 or 22 carbon atoms, more preferably selected from the group consisting of methyl, ethyl, propyl, butyl, pentyl, hexyl, heptyl, octyl, nonyl, decyl, undecyl, dodecyl, tridecyl, pentadecyl, heptadecyl, eicosyl and docosyl. In another preferred embodiment in combination with any of the above or below embodiments, R2 is an optionally substituted C6-14 alkyl, in particular, 10-hydroxydecyl.

In another preferred embodiment in combination with any of the above or below embodiments, R2 is an optionally substituted C2-50 alkenyl group, preferably an optionally substituted C2-22 alkenyl group, i.e. an alkenyl group containing 2, 3, 4, 5, 6, 7, 8, 9, 10, 11, 12, 13, 14, 15, 16, 17, 18, 19, 20, 21 or 22 carbon atoms, more preferably, an optionally substituted C2-15 alkenyl group, in particular, selected from the group consisting of ethenyl, 1-propenyl, 3-methyl-2-butenyl and 3,7-dimethyl-2,6-octadienyl. The number of double bonds may generally range from 1 to 3 and these double bonds may be conjugated.

In another preferred embodiment in combination with any of the above or below embodiments, R2 is an alkyl or alkenyl group, wherein the alkyl or alkenyl is substituted by 1 to 10 substituents, more preferably 1 to 3 substituents, selected from the group consisting of (i) C1-4 alkyl, (ii) hydroxy, (iii) oxo, (iv) amino, (v) mono-C1-6 alkylamino, (vi) di-C1-6 alkylamino, (vii) carboxy, (viii) C1-4 alkoxycarbonyl, (ix) C6-14 aryl, which may be substituted by 1 or 2 substituents selected from the group consisting of C1-4 alkyl, hydroxy, carboxy and C1-6 alkoxycarbonyl, (x) 5- or 6-membered heterocyclic group, which may be substituted by 1 or 2 substituents selected from the group consisting of C1-4 alkyl, hydroxy, carboxy and C1-6 alkoxycarbonyl, and (xi) halogen; and wherein R2 is more preferably a C6-14 alkyl substituted by hydroxy. The C1-4 alkoxycarbonyl group is preferably methoxycarbonyl, ethoxycarbonyl, propionyloxycarbonyl or butoxycarbonyl. The aryl is preferably phenyl, 1-naphthyl, 2-naphthyl or indanyl. The heterocyclic group is preferably 2-pyridyl, 3-pyridyl, 2-thienyl or 3-thienyl. The halogen can be fluorine, chlorine, bromine or iodine.

In another preferred embodiment in combination with any of the above or below embodiments, R2 is a substituted alkyl or substituted alkenyl group, wherein the substituent is a substituted aryl or a substituted heterocyclic group, and wherein the substituent on the aryl or heterocyclic group is 1 or 2 substituents selected from a C1-4 alkyl group, hydroxy, carboxy, and C1-5 alkoxycarbonyl. The C1-4 alkyl group is preferably methyl, ethyl, propyl or butyl. The C1-5 alkoxycarbonyl is preferably methoxycarbonyl, ethoxycarbonyl, propoxycarbonyl or butoxycarbonyl. The one or more substituents on the aryl or heterocyclic group can be present in any position of the ring structure.

In another preferred embodiment in combination with any of the above or below embodiments, R2 is a substituted alkyl or substituted alkenyl group, wherein the position of substitution on the alkyl or alkenyl group is the 1-position or omega-position.

In another preferred embodiment in combination with any of the above or below embodiments, each of R3 and R4 represents independently a C1-6 alkyl, preferably C1-4 alkyl, the alkyl being optionally substituted by 1 to 3 substituents selected from the group consisting of hydroxy, halogen, nitro, C1-3 alkyl, C1-3 haloalkyl, carboxy, C1-6 alkoxycarbonyl, 3-pyridyl, 1-imidazolyl and 5-thiazolyl; a C1-3 alkoxy, preferably methoxy; or R3 and R4 taken together represent a butadienylene group optionally substituted by 1 to 3 substituents selected from the group consisting of C1-3 alkyl, C1-3 alkoxy, hydroxy, nitro and halogen.

In another preferred embodiment in combination with any of the above or below embodiments, R3 is an optionally substituted C1-6 alkyl group, i.e. an alkyl group containing 1, 2, 3, 4, 5, or 6 carbon atoms, more preferably selected from methyl, ethyl, propyl, i-propyl, butyl, i-butyl, sec-butyl, t-butyl, amyl and hexyl. More preferably, R3 is an optionally substituted C1-4 alkyl group.

In another preferred embodiment in combination with any of the above or below embodiments, R3 is a substituted alkyl group, which is substituted by 1 to 3 substituents selected from the group consisting of hydroxy, halogen, nitro, C1-3 alkyl, C1-3 haloalkyl, carboxy, C1-6 alkoxycarbonyl, 3-pyridyl, 1-imidazolyl and 5-thiazolyl; a C1-3 alkoxy, preferably methoxy. The C1-3 haloalkyl is preferably trifluoromethyl. The C1-6 alkoxycarbonyl is preferably C1-5 alkoxycarbonyl, more preferably methoxycarbonyl or ethoxycarbonyl. The halogen can be fluorine, chlorine, bromine or iodine.

In another preferred embodiment in combination with any of the above or below embodiments, R3 is a C1-3 alkoxy group, i.e. an alkoxy group containing 1, 2 or 3 carbon atoms, more preferably methoxy, ethoxy, propoxy or i-propoxy, in particular, methoxy.

In another preferred embodiment in combination with any of the above or below embodiments, R4 is an optionally substituted C1-6 alkyl group, i.e. an alkyl group containing 1, 2, 3, 4, 5, or 6 carbon atoms, more preferably, selected from methyl, ethyl, propyl, i-propyl, butyl, i-butyl, sec-butyl, t-butyl, amyl and hexyl. More preferably, R4 is an optionally substituted C1-4 alkyl group.

In another preferred embodiment in combination with any of the above or below embodiments, R4 is a substituted alkyl group, which is substituted by 1 to 3 substituents selected from the group consisting of hydroxy, halogen, nitro, C1-3 alkyl, C1-3 haloalkyl, carboxy, C1-6 alkoxycarbonyl, 3-pyridyl, 1-imidazolyl and 5-thiazolyl; a C1-3 alkoxy, preferably methoxy. The C1-3 haloalkyl is preferably trifluoromethyl. The C1-6 alkoxycarbonyl is preferably C1-5 alkoxycarbonyl, more preferably methoxycarbonyl or ethoxycarbonyl. The halogen can be fluorine, chlorine, bromine or iodine.

In another preferred embodiment in combination with any of the above or below embodiments, R4 is a C1-3 alkoxy group, i.e. an alkoxy group containing 1, 2 or 3 carbon atoms, more preferably methoxy, ethoxy, propoxy or i-propoxy, in particular, methoxy.

In another preferred embodiment in combination with any of the above or below embodiments, R3 and R4 taken together represent an optionally substituted butadienylene group. Thus, R3 and R4 together with the carbon atoms to which they are attached form a benzene ring. Preferably, this benzene ring is substituted by 1 to 3 substituents selected from a C1-3 alkyl group, a C1-3 alkoxy group, hydroxy, nitro and halogen. The C1-3 alkyl groups are preferably methyl, ethyl or propyl. The C1-3 alkoxy groups are preferably methoxy, ethoxy or propoxy.

The term "alkyl" designates linear or branched alkyl, wherein the number of carbon atoms is as specified herein.

The term "alkenyl" designates linear or branched alkenyl, wherein the number of carbon atoms is as specified herein.

The term "aryl" designates an aromatic carbocyclic group containing from 6 to 22 atoms, more preferably, 6 to 14 carbon atoms, such as phenyl or naphthyl.

The term "heterocyclic group" designates a saturated or partically unsaturated group containing from 1 to 14 carbon atoms and from 1 to 4 atoms selected from N, O or S.

In a preferred embodiment in combination with any of the above or below embodiments, the active ingredient of formula (I) is idebenone or an idebenone analogue, preferably idebenone.

The term "idebenone analogues", as used herein, encompasses natural ubiquinones (coenzyme Q-n) as well as their structural analogs having a reversibly reducible quinone ring with a lipophilic side chain, for example, decylubiquinone.

The term "thin film drug delivery system" as used herein designates a delivery system wherein the active ingredient is embedded in a matrix of polymeric carrier material and which is significantly thinner than other known delivery systems, such as tablets or pastilles. Usually, the thin film has an area of between 1 and 10 cm², a weight per unit area between 50 and 250 g/m² and a thickness of between 40 and 300 µm. The thin film drug delivery system is also designated as oral wafer.

In a preferred embodiment in combination with any of the above or below embodiments, the carrier material is selected from the group consisting of cellulose or a derivative thereof, a polyvinyl alcohol (PVA), poly-N-vinylpyrrolidone, a vinyl-pyrrolidone-vinyl acetate copolymer, starch, a starch derivative, gelatin, a gelatin derivative, and a combination thereof, more preferably selected from the group consisting of a cellulose derivative, a polyvinyl alcohol, a vinyl-pyrrolidone-vinyl acetate copolymer, a starch derivative, a gelatin derivative, and a combination thereof, even more preferably selected from the group consisting of a cellulose derivative, a polyvinyl alcohol, a vinyl-pyrrolidone-vinyl acetate copolymer, and a combination thereof.

In another preferred embodiment in combination with any of the above or below embodiments, the carrier material is a cellulose derivative selected from the group consisting of methylcellulose, ethylcellulose, hydroxypropyl-cellulose, hydroxyethylcellulose, hydroxypropylmethyl-cellulose (HPMC), carboxymethylcellulose (CMC) and a combination thereof, more preferably selected from the group consisting of ethylcellulose, hydroxypropyl-cellulose, hydroxyethylcellulose, hydroxypropylmethyl-cellulose (HPMC), carboxymethyl cellulose (CMC) and a combination thereof, even more preferably selected from hydroxypropylmethyl-cellulose (HPMC) and carboxymethylcellulose (CMC).

In another preferred embodiment in combination with any of the above or below embodiments, the carrier material is a starch derivative selected from the group consisting of hydroxypropyl starch and sodium starch glycolate.

In another preferred embodiment in combination with any of the above or below embodiments, the thin film drug delivery system comprises 0.01 to 80% by weight, more preferably, 2-70% by weight of active ingredient.

In another preferred embodiment in combination with any of the above or below embodiments, the thin film drug delivery system comprises 20-99.99% by weight, more preferably, 30-98% by weight of a carrier material.

In a preferred embodiment in combination with any of the above or below embodiments, the system comprises a carrier matrix, wherein the active ingredient, preferably idebenone, is incorporated as a suspension, a suspension after micronization, an emulsion, a micro- or nano-emulsion, or in solubilised form (mono-molecular dispersion), more preferably in solubilised form (mono-molecular dispersion).

In another preferred embodiment in combination with any of the above or below embodiments, the thin film drug delivery system comprises a suspension of 30-60% by weight, more preferably 40-50% by weight, of active ingredient and 40-70% by weight, more preferably 50-60% by weight of a carrier material. The active ingredient is preferably present in micronized form in the suspension. The carrier material is preferably a polyvinyl alcohol and/or a cellulose derivative.

In another preferred embodiment in combination with any of the above or below embodiments, the thin film drug delivery system comprises 3-20% by weight, more preferably 5-10% by weight, of a solubilised form (mono-molecular dispersion) of the active ingredient and 80-97% by weight, more preferably 90-95% by weight, of a carrier material. The carrier material is preferably a substituted carbohydrate or any other water soluble polymer. The substituted carbohydrate is preferably a cellulose derivative or a starch derivative.

In another preferred embodiment in combination with any of the above or below embodiments, the thin film drug delivery system comprises 3-50% by weight, more preferably 5-30% by weight, of an emulsion of the active ingredient and 50-97% by weight, more preferably 70-95% by weight, of a carrier material. The carrier material is preferably a cellulose derivative.

In another preferred embodiment in combination with any of the above or below embodiments, the thin film drug delivery system comprises 50% by weight of an active ingredient selected from the group consisting of idebenone or an idebenone analogue, more preferably idebenone, 40% by weight of poly-vinyl alcohol (PVA) and 10% by weight of sodium carboxymethyl-cellulose carrier material.

In another preferred embodiment in combination with any of the above or below embodiments, the thin film drug delivery system comprises 10% by weight of an active ingredient selected from the group consisting of idebenone or an idebenone analogue, more preferably idebenone, and 90% by weight of HPMC carrier material.

In another preferred embodiment in combination with any of the above or below embodiments, the thin film drug delivery system comprises one or more further substances selected from the group consisting of flavorings, colorants, sweeteners, fillers, plasticizers, surface-active substances, liquid--preferably lipophilic--excipients which are able to dissolve the active ingredient and form a second phase in the--preferably hydrophilic--carrier material, solubilizers, pH stabilizers, disintegrants, solubility enhancers, absorption enhancers, and/or permeation enhancers.

In another preferred embodiment in combination with any of the above or below embodiments, the thin film drug delivery system comprises at least one excipient from the group group consisting of flavorings, colorants, sweeteners, fillers, plasticizers, surface-active substances, solubilizers, liquid excipient, pH stabilizers, disintegrants, solubility enhancers and absorption enhancers.

In another preferred embodiment in combination with any of the above or below embodiments, the thin film drug delivery system comprises an active ingredient which is micronized. Micronisation involves the reduction of particles to a size of preferably less than 100 µm, more preferably less than 10 µm.

In another preferred embodiment in combination with any of the above or below embodiments, the thin film drug delivery system of the present invention has an area of between 1 and 10 cm², more preferably, between 2 and 8 cm², and, in particular, between 5 and 7 cm².

In another preferred embodiment in combination with any of the above or below embodiments, the thin film drug delivery system of the present invention has a weight per unit area between 50 and 250 g/m², more preferably, between 100 and 150 g/m².

In another preferred embodiment in combination with any of the above or below embodiments, the thin film drug delivery system of the present invention has a thickness of between 40 and 300 µm, more preferably, between 50 and 100 µm.

The thin film drug delivery system has preferably a monolayer or double-layer construction, more preferably a monolayer construction.

In a preferred embodiment in combination with any of the above or below embodiments, the thin film drug delivery system is for use in the treatment of a mitochondrial disease, preferably selected from the group consisting of Leber's hereditary optic neuropathy (LHON), autosomal dominant optic atrophy (DOA), macular degeneration, glaucoma, retinopathy, cataract, optic disc drusen (ODD), mitochondrial myopathy, encephalomyopathy, lactic acidosis, stroke-like symptoms (MELAS), myoclonic epilepsy with ragged red fibers (MERRF), myoneurogenic gastrointestinal encephalomyopathy (MNGIE), Kearns-Sayre syndrome, CoQ10 deficiency, and mitochondrial complex deficiencies (1-5, CPEO);
a neurodegenerative disease, preferably selected from the group consisting of Friedreich's ataxia (FRDA), amyotrophic lateral sclerosis (ALS), Parkinson's disease, Alzheimer's disease, Huntington's disease, stroke/reperfusion injury, and dementia;
a neuromuscular disease, preferably selected from the group consisting of Duchenne muscular dystrophy (DMD), Becker muscular dystrophy (BMD), Limb-Girdle muscular dystrophy (LGMD), X-linked dilated cardiomyopathy (XLDCM), Pantothenate kinase-associated neurodegeneration (PKAN), spinal muscular atrophy (SMA), multiple sclerosis and primary progressive multiple sclerosis (PP-MS), Kugelberg-Welander disease, and Werdnig-Hoffmann disease;
a psychiatric disorder, preferably selected from the group consisting of schizophrenia, major depressive disorder, bipolar disorder, and epilepsy;
a metabolic disorder, preferably selected from the group consisting of ageing-related physical decline, obesity, overweight, type II diabetes, and metabolic syndrome;
cancer; multiple sclerosis; primary progressive multiple sclerosis; or
immune dysfunction, preferably selected from the group consisting of arthritis, psoriasis and rheumatoid arthritis.

In a preferred embodiment in combination with any of the above or below embodiments, the thin film drug delivery system is for use in the treatment of a mitochondrial disease. Preferably, the mitochondrial disease is selected from the group consisting of Leber's hereditary optic neuropathy (LHON), autosomal dominant optic atrophy (DOA), macular degeneration, glaucoma, retinopathy, cataract, optic disc drusen (ODD), mitochondrial myopathy, encephalomyopathy, lactic acidosis, stroke-like symptoms (MELAS), myoclonic epilepsy with ragged red fibers (MERRF), myoneurogenic gastrointestinal encephalomyopathy (MNGIE), Kearns-Sayre syndrome, CoQ10 deficiency, and mitochondrial complex deficiencies (1-5, CPEO).

In a preferred embodiment in combination with any of the above or below embodiments, the thin film drug delivery system is for use in the treatment of Leber's hereditary optic neuropathy (LHON).

In a preferred embodiment in combination with any of the above or below embodiments, the thin film drug delivery system is for use in the treatment of mitochondrial myopathy, encephalomyopathy, lactic acidosis, stroke-like symptoms (MELAS).

In a preferred embodiment in combination with any of the above or below embodiments, the thin film drug delivery system is for use in the treatment of a neurodegenerative disease. Preferably, the neurodegenerative disease is selected from the group consisting of Friedreich's ataxia (FRDA), amyotrophic lateral sclerosis (ALS), Parkinson's disease, Alzheimer's disease, Huntington's disease, stroke/reperfusion injury, and dementia.

In a preferred embodiment in combination with any of the above or below embodiments, the thin film drug delivery system is for use in the treatment of a neuromuscular disease.

Preferably, the neuromuscular disease is selected from the group consisting of Duchenne muscular dystrophy (DMD), Becker muscular dystrophy (BMD), Limb-Girdle muscular dystrophy (LGMD), X-linked dilated cardiomyopathy (XLDCM), Pantothenate kinase-associated neurodegeneration (PKAN), spinal muscular atrophy (SMA), multiple sclerosis and primary progressive multiple sclerosis (PP-MS), Kugelberg-Welander disease, and Werdnig-Hoffmann disease.

In a preferred embodiment in combination with any of the above or below embodiments, the thin film drug delivery system is for use in the treatment of Duchenne muscular dystrophy (DMD).

In a preferred embodiment in combination with any of the above or below embodiments, the thin film drug delivery system is for use in the treatment of primary progressive multiple sclerosis (PP-MS).

In a preferred embodiment in combination with any of the above or below embodiments, the thin film drug delivery system is for use in the treatment of a psychiatric disorder. Preferably, the psychiatric disorder is selected from the group consisting of schizophrenia, major depressive disorder, bipolar disorder, and epilepsy.

In a preferred embodiment in combination with any of the above or below embodiments, the thin film drug delivery system is for use in the treatment of a metabolic disorder. Preferably, the metabolic disorder is selected from the group consisting of ageing-related physical decline, obesity, overweight, type II diabetes, and metabolic syndrome.

In a preferred embodiment in combination with any of the above or below embodiments, the thin film drug delivery system is for use in the treatment of cancer.

In a preferred embodiment in combination with any of the above or below embodiments, the thin film drug delivery system is for use in the treatment of multiple sclerosis.

In a preferred embodiment in combination with any of the above or below embodiments, the thin film drug delivery system is for use in the treatment of primary progressive multiple sclerosis.

In a preferred embodiment in combination with any of the above or below embodiments, the thin film drug delivery system is for use in the treatment of immune dysfunction. Preferably, the immune dysfunction is selected from the group consisting of arthritis, psoriasis and rheumatoid arthritis.

Suitable doses of the active ingredient administered by thin film formulation (oral wafer) are 0.01 mg/kg/day to 60 mg/kg/day. Preferably, for example, idebenone is administered in a dosage of 0.01 mg/kg/day to 20 mg/kg/day, more preferably, in a dosage of 0.01 mg/kg/day to 10 mg/kg/day and even more preferably, in a dosage of 0.01 mg/kg/day to less than 5 mg/kg/day. Most preferably, the dosage of the active ingredient idebenone is between 0.1 mg/kg/day to 4 mg/kg/day. Studies have shown that, surprisingly, such low dosages achieve the required plasma level of idebenone if it is applied via the oral mucosa. The required dosage may be ascertained readily by a person skilled in the art.

In another preferred embodiment in combination with any of the above or below embodiments, the active ingredient, preferably idebenone, is administered in combination with a second therapeutic agent, wherein said second therapeutic agent is preferably selected from glucocorticosteroids, in particular, 6a-methylprednisolone-21 sodium succinate (solumedrol^{®}) or deflazacort (calcort^{®}), which are routinely used in DMD patients for treatment of muscle weakness.

In another preferred embodiment in combination with any of the above or below embodiments, the active ingredient, preferably idebenone, is administered in combination with any medicament used in DMD patients to treat DMD-associated cardiomyopathy such as ACE-inhibitors, beta-blockers and diuretics as well as HMG-CoA reductase inhibitors.

In another preferred embodiment in combination with any of the above or below embodiments, the active ingredient, preferably idebenone, is administered in combination with a further therapeutic agents, more preferably, erythropoietin, vitamin E or vitamin C.

In another preferred embodiment in combination with any of the above or below embodiments, the active ingredient, preferably idebenone, and a further therapeutic agent can be used simultaneously, separately or sequentially in order to treat or prevent the disease symptoms. The therapeutic agents may be provided in a single dosage form or as separate formulations, each formulation containing at least one of the active agents.

The present invention is also directed to a method for preparing the thin film drug delivery system, comprising
a) dissolving or dispersing the active ingredient and the carrier in a solvent;
b) drying the solution or dispersion of active ingredient and carrier in order to obtain a thin film.

In a preferred embodiment in combination with any of the above or below embodiments, the method comprises a) dissolving the active ingredient and the carrier in a solvent.

In a preferred embodiment in combination with any of the above or below embodiments, the solvent is selected from the group consisting of methanol, ethanol, isopropanol, aceton, cyclohexane, water and a combination thereof.

In a preferred embodiment in combination with any of the above or below embodiments, the method comprises degassing the solution of active ingredient and carrier.

In a preferred embodiment in combination with any of the above or below embodiments, the method comprises casting/spreading out the solution of active ingredient and carrier.

In a preferred embodiment in combination with any of the above or below embodiments, the method comprises cutting the film into individual units.

The manufacturing process typically leads to large sheets with the active ingredients homogeneously incorporated in the polymer matrix as a carrier. Individual units are subsequently packed in pharmaceutically acceptable configurations.

Depending on the type of thin film formulation, it is possible to generate different pharmacokinetic profiles and avoid undesirable side effects in the gastro-intestinal tract. The drug load per unit largely depends on the physicochemical properties of the drug.

This type of formulation is intended to be applied to the oral mucosa where the active ingredient is absorbed into the body through the mucosa. According to the invention, such formulation constitutes the basis of a medicament containing idebenone for this specific route of administration. The oral mucosa comprises the mucosa in the entire oral and connecting cavities, including, but not limited to, the sublingual, buccal, gingival, lingual, as well as the esophageal mucosa.

In a preferred embodiment in combination with any of the above or below embodiments, the system is administered via the oral mucosa on or under the tongue in the buccal cavity or any other location in the oral cavity.

The administration system releases the active ingredient contained therein with a high bioavailability. Preferably, the system will be able to achieve a high bioavailability without the addition of permeation enhancers.

The inventive thin film drug delivery system can, in a particular embodiment, be designed such that it has mucoadhesive properties. Mucoadhesive properties may be obtained by addition of mucoadhesive polymers, preferably selected from the group consiting of hyaluronic acid, poly(acrylic acid) derivatives, cellulose derivatives, in particular sodium carboxymethyl cellulose or hydroxylethyl cellulose, xanthan gum, guar gum, carrageenan, sodiumalginate, chitosan, poly (vinyl alcohol) or polycarbophil. By this, it is meant that the property of adhering to a mucous membrane, specifically in such a manner that it is difficult or impossible to detach the administration system from the mucous membrane subsequent to the application.

In a preferred embodiment in combination with any of the above or below embodiments, the system is a mucoadhesive film which dissolves in the mouth.

It is also preferred that the administration system is in or forms a gel-like consistency in the oral cavity upon swelling in saliva.

The thin film drug delivery system dissolves in the mouth preferably in a period of less than 30 min, more preferably in a period of less than 15 min.

The active ingredient which enters the bloodstream transmucosally from the administration system leads to a rapid rise in the concentration of this active ingredient in the blood. In this case, a maximum concentration of the active ingredient in the blood is reached preferably in a period of less than 60 min, more preferably, in a period of between 5 and 30 min after application.

With the thin film drug delivery system of the present invention it is possible to achieve a relatively high bioavailability, as measured by the AUC of the active ingredient concentration in the blood, of at least a factor of 5 times (500%) greater, preferably, of at least a factor of 10 (1000%) greater than oral administration, when adjusted for the dose administered. A particularly preferred increase in bioavailability would be a factor of 20 (2000%) greater.

The active ingredient, especially idebenone (International Nonproprietary Name (INN): idebenone; Chemical name: 2-(10-Hydroxydecyl)-5,6-dimethoxy-3-methyl-cyclohexa-2,5-diene-1,4-dione; Chemical Abstracts Service (CAS) registry number: 58186-27-9) and its analogues are provided in the form of a thin film formulation (also called oral wafer) that when attached to the oral mucosa, releases the active ingredient directly to the mucosa or partly into the saliva in the oral cavity and is used for transmucosal administration to human beings or animals. This type of system results in much higher plasma levels of the compound compared to the oral route of administration.

Idebenone has the following chemical structure formula (II): 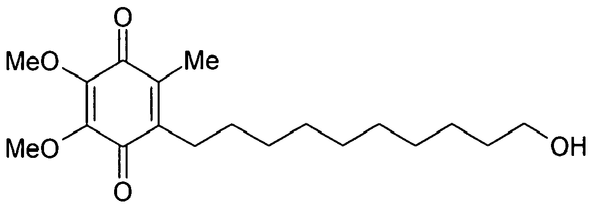 2-(10-Hydroxydecyl)-5,6-dimethoxy-3-methyl-cyclohexa-2,5-diene-1,4-dione, idebenone

Idebenone has been tested thoroughly in toxicological animal studies and has been made the subject of various medical studies investigating its efficacy in the treatment of, for example, neuromuscular diseases such as Friedreich's ataxia, Duchenne muscular dystrophy or neurological diseases such as Alzheimer's disease. Therefore, idebenone may be regarded as toxicologically safe, which means that it can be used as a pharmaceutical active agent in a medicament. The toxicological safety of idebenone has been confirmed in several clinical studies in several indications:
■ Alzheimer's disease (AD) with 536 patients that have been treated with up to 360 mg of idebenone t.i.d. (ter in die) = 1080 mg/day. (L. J. Thal, M. Grundman, J. Berg, K. Ernstrom, R. Margolin, E. Pfeiffer, M. F. Weiner, E. Zamrini, R. G. Thomas, Neurology 61 (2003), 1498-1502).
■ Duchenne muscular dystrophy (DMD) with 21 patients and doses up to 150 mg t.i.d. (Buyse GM, Goemans N, van den Hauwe M, Thijs D, de Groot IJ, Schara U, Ceulemans B, Meier T, Mertens L. Idebenone as a novel, therapeutic approach for Duchenne muscular dystrophy: results from a 12 month, double-blind, randomized placebo-controlled trial. Neuromuscul Disord 2011;21:396-405)
■ Friedreich's ataxia (FRDA) in 3 studies with 70, 48 and 78 patients and doses up to 750 mg t.i.d. (Lynch DR, Perlman SL, Meier T. A Phase 3, double-blind, placebo-controlled trial of idebenone in Friedreich ataxia. Arch Neurol 2010;67:941-47; Di Prospero NA, Baker A, Jeffries N, Fischbeck KH. (2007) Neurological effects of high-dose idebenone in patients with Friedreich's ataxia: a randomised, placebo-controlled trial. Lancet Neurol. 6:878-86; Di Propsero N.A., Sumner C.J., Penzak S.R., Ravina B., Fischbeck K.H., Taylor J.P. (2007 a). Safety, tolerability, and pharmacokinetics of high-dose idebenone in patients with Friedreich ataxia. Arch Neurol 64; 803-808)
■ Leber's Hereditary Optic Neuropathy (LHON) with 85 patients and doses up to 300 mg t.i.d. (Klopstock T, Yu-Wai-Man P, Dimitriadis K. et al. A randomized placebo-controlled trial of idebenone in Leber's hereditary optic neuropathy. 2011 Brain 134: 2677-86)

It has now been observed that, after conventional oral administration and absorption in the gut, idebenone is rapidly metabolized during its first passage through the liver. The major metabolites are idebenone conjugates such as glucuronates and sulphates as well as derivatives where the side chain of the parent compound has been oxidized. The metabolites of idebenone are pharmacologically not significantly active and they are rapidly excreted. Due to this strong first pass metabolism, oral administration of idebenone requires high doses in order to reach pharmacologically active plasma levels. These high doses result in unwanted effects such as diarrhea and gastrointestinal (GI) tract disturbances which are frequently observed in clinical applications.

Using the thin film formulation (oral wafer), it has surprisingly been found that even with a 20-fold lower dose compared to the oral administration route, a more than 5-fold AUC can be achieved, which, on a dose-normalized level, leads to a >100-fold AUC (see Table 4). Moreover, this opens up possibilities to achieve plasma levels of the pharmacologically active molecule that are far above those achievable via the oral route of administration.

By use of this system and the transmucosal route of administration, the high first pass metabolism observed after conventional oral administration of idebenone can very effectively be circumvented.

The thin film drug delivery system of the present invention shows favorable properties with regard to patient convenience and a surprisingly much higher effect on plasma levels than described e.g. in WO-A-2008/019769. The area under the curve (AUC) of idebenone plasma levels on a dose normalized basis was improved by a factor of 11 in the example described in WO-A-2008/019769, whereas the examples of the present invention improved the dose normalized AUC by a factor of 26 - 121.

Thus, the best effect of improved plasma levels by circumventing the first-pass-effect can be achieved by the inventive thin film (oral wafer) formulation. The thin film drug delivery system of the present invention comprises an active ingredient embedded in the form of particles, preferably micronized particles, as oil droplets or as monomolecular dispersion, in the polymeric carrier material. The oil droplets have preferably a size of 0.5 to 50 µm, more preferably 0.5 to 5 µm.

A thin film formulation is a solid form which includes most advantages of classical solid forms like tablets or capsules, such as chemical/physical stability and dosing accuracy, but is still fundamentally different to a tablet or capsule and has a number of additional advantages.

Compared to conventional oral formulations, such as tablets, the use of the oral wafer formulation of idebenone leads to:
a) significant reduction of the dose that has to be administered whilst similarly high or higher plasma levels of this active ingredient are obtained. Lower active ingredient exposure is generally believed to be associated with a reduced risk of adverse side-effects and offers a medical advantage leading to improved compliance by the patient. In the particular case of idebenone, the described GI side effects can be avoided.
b) significantly higher plasma levels compared to those achievable with an oral formulation of the active ingredient being absorbed via the gastrointestinal route. This may lead to extension of the use of idebenone to additional, new indications that require high concentrations e.g. in order to cross the blood-brain-barrier.
c) increased patient convenience as the formulation is flat, easy to handle and does not require any water for administration. Moreover, one unit is capable of replacing several tablets.
d) lower food effect, which is strongly pronounced when idebenone is administered via the oral route and leads to instructions to use the oral tablets only with a meal. The oromucosal route is independent of food intake and therefore reduces variability of plasma levels.
e) enabling administration to patients with swallowing difficulties, such as patients suffering from certain neuromuscular and neurological diseases, elderly patients or children below the age of 8, resulting in increased compliance.

Compared to the efficacious dose of idebenone administered via the conventional oral route of administration and absorption in the gastrointestinal tract, the efficacious dose of the thin film drug delivery system of the present invention is significantly lower. This delivery system leads to significantly higher plasma levels of the active ingredient and the achievement of increased clinical efficacy. Moreover, due to the higher plasma levels, the use of idebenone can be extended to additional medical fields, particularly those which require high concentrations of drug substance e.g. in order to cross the blood-brain-barrier.

The following examples illustrate the invention.

### EXAMPLE 1

112 g of PVA was added to 720 mL of water and stirred until dissolution was complete. The dissolution was assisted by application of heat. After cooling, 140 g of idebenone was dispersed uniformly. Afterward, 28 g of CMC was added, and the mixture was stirred until dissolution was complete.

The mixture was degassed, spread out with the aid of a spreading box and dried. A thin opaque film which was between 50 and 150 µm thick was produced. Opaque wafers with a content of 30 mg of idebenone were obtained by cutting out samples of the appropriate size.

### EXAMPLE 2

28 g of idebenone were added to 720 mL of 75:25 methanol:water, which was stirred until the active ingredient had completely dissolved. 250 g of HPMC were added and stirred until dissolution is complete. Degassing of the mixture and spreading out were followed by drying. A thin translucent film which was between 100 and 300 µm thick was produced. Translucent wafers with a content of 15 mg of idebenone were obtained by cutting out samples of the appropriate size.

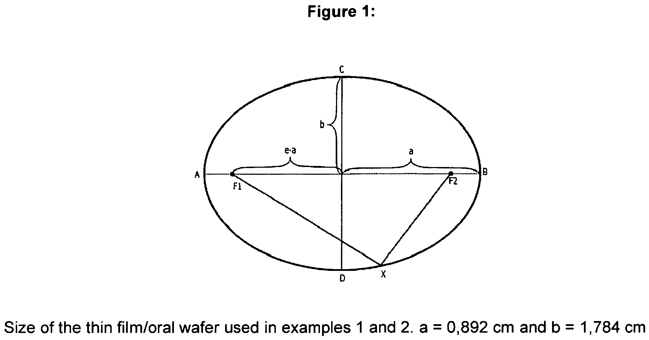

The size of the thin film/oral wafer used in examples 1 and 2 is shown in Figure 1, wherein a = 0,892 cm and b = 1,784 cm leading to a total area of 5.00 cm².

The dried system of example 1 comprises the active ingredient as particles in a separate phase suspended in the carrier material, whereas the active ingredient in example 2 is in the form of a mono-molecular dispersion in the carrier material.

### Experimental Data:

### Pharmacokinetic data after oromucosal delivery of idebenone

Plasma levels of idebenone were studied after the administration of two different thin film formulations (oral wafers) administered via the oromucosal route in dogs and compared to the profile obtained when idebenone is dosed by the oral route (gavage) in the form of a micro-emulsion. The doses used in this study were 30 mg oromucosal (oral wafer A, suspension type = example 1), 15 mg oromucosal (oral wafer B, mono-molecular dispersion type = example 2) and 300 mg oral (TPGS/Miglyol microemulsion administered by oral gavage). The TPGS/Miglyol microemulsion consists of 0.400 g idebenone, 2.880 g Miglyol 812N, 1.920 g d-alpha tocopheryl polyethylene glycol 1000 succinate (TPGS) and 15 g water. The study was a three-way cross-over study with one week wash out between administrations. Formulations were administered to female Beagle dogs under fasting conditions. The pharmacokinetic exposure of parent (unconjugated) idebenone and the total of its metabolites (total idebenone, total QS10, total QS6, and total QS4) was determined for each formulation.

Blood samples were collected at several time points over 6 hours after administration. Concentrations of idebenone in plasma were measured by HPLC-MS/MS and pharmacokinetic parameters were calculated.

For this, idebenone was separated and quantified by HPLC-MS/MS: For HPLC, a Synergi™ 4µ MAX-RP (50 x 2 mm) column (Phenomenex, Schlieren, Switzerland) was used. Column temperature: 50°C. Mobile phase A: water + 1 M NH₄Oac solution (pH4, 100 + 3, v+v); mobile phase B: MeOH/H₂O 100/3 (v/v) + 1M NH₄Oac solution (pH4, 100 + 3, v/v), gradient elution (table 4). Flow: 250 µl/min and 400µl/min.

Once separated, idebenone was quantified by ESI-MS/MS (API 4000, Perkin-Elmer-Europe BV, Rotkreuz, Switzerland) in positive mode.

**Table 1: Pump-gradient program and time events for separation and quantification of idebenone**

| Time [min] | mobile phase B [%] | Flow [µl/min] | Comments |
|---|---|---|---|
| 0.01 | 40 | 250 | start gradient, HPLC eluent to waste |
| 0.04 | - | 250 | Start MS |
| 1.00 | - | 250 | HPLC eluent to MS |
| 3.50 | - | 250 | - |
| 3.51 | - | 400 | - |
| 3.75 | 95 | 400 | - |
| 4.50 | 95 | 400 | - |
| 4.51 | 40 | 400 | - |
| 5.50 | 40 | 400 | HPLC eluent to waste |
| 5.90 | 40 | 400 | - |
| 5.91 | 40 | 250 | - |
| 6.00 | 40 | 250 | End of run |
| 29.99 | 40 | 250 | Pump shutdown |
| 30.00 | 95 | 20 | |

From times 0.01 to 3.75 min, a linear gradient was used.

Idebenone conjugates such as glucuronates and sulphates have been quantified after acidic hydrolysis as described by R. Artuch, C. Colomé, M. A. Vilaseca, A. Aracil. M. Pineda, J. Neurosci. Meth. 115 (2002), 63-66.

The pharmacokinetic analysis included maximum plasma concentration (Cₘₐₓ), the time when maximum plasma concentration was observed (Tₘₐₓ), and the area under the plasma concentrations versus time curve from time 0 h to 360 min (AUC₀₋₃₆₀ₘᵢₙ). The relative bioavailability of idebenone after sublingual administration compared to the oral administration was calculated for each dog from normalized (1 mg/kg) AUC values. The AUC ratios of the metabolites were also calculated. In addition, Cₘₐₓ ratios, normalized to a 1 mg/kg dose, were calculated.

The results obtained are shown in Table 2 to below.

**Table 2: Mean pharmacokinetic parameters of idebenone after oromucosal (30 mg wafer A or 15 mg wafer B) vs. oral (300 mg, microemulsion) administration in dogs.**

| Dosing | Cₘₐₓ | Tₘₐₓ | AUC₀₋₃₆₀ |
|---|---|---|---|
| | [ng/ml] | [min] | [min*ng/ml] |
| 30 mg wafer A; example 1 | | | |
| (micronized suspension) | 248 | 15 | 6152 |
| 15 mg wafer B, example 2 | | | |
| (mono-molecular dispersion) | 404 | 25 | 13864 |
| 300 mg oral gavage | | | |
| (microemulsion) | 111 | 7 | 2305 |

As shown in Table 2, the two wafer formulations of idebenone administered via the oromucosal route, prepared according to example 1 and 2, clearly lead to significantly higher plasma levels of idebenone compared to conventional oral administration. Both Cₘₐₓ and AUC₀₋₃₆₀ were superior to the oral administration in both thin film (oral wafer) formulations. There is also a marked difference between wafer A, which contains 30 mg idebenone as micronized powder suspended in the polymer matrix, and wafer B, which contains only 15 mg but in a solid solution, i.e. molecularly dispersed state. The latter obviously dissolves much more efficiently and is better absorbed by the oral mucosa.

Figure 2 shows the mean plasma concentrations versus time of free idebenone after single administrations of various formulations (oral gavage and thin film/oral wafers) in female Beagle dogs. Wafer B (15 mg, solid solution type wafer) shows the highest Cₘₐₓ and the largest AUC compared to the other treatment arms. Wafer A (30 mg, suspension type wafer) shows a higher Cₘₐₓ and a larger AUC compared to the oral route (300 mg, microsuspension).

**Table 3: Mean pharmacokinetic parameters of idebenone after oromucosal (30 mg wafer A or 15 mg wafer B) vs. oral (300 mg, microemulsion) administration in dogs after dose normalization per mg.**

| Dosing | Cₘₐₓ / mg | AUC₀₋₃₆₀ / mg |
|---|---|---|
| | [ng/ml] | [min*ng/ml] |
| 30 mg wafer A; example 1 | | |
| (micronized suspension) | 8.3 | 205 |
| 15 mg wafer B; example 2 | | |
| (mono-molecular dispersion) | 26.9 | 924 |
| 300 mg oral gavage | | |
| (microemulsion) | 0.4 | 7.7 |

As shown in Table 3: above, the magnitude of increase in exposure obtained with the two wafer formulations of idebenone administered via the oromucosal route (prepared according to example 1 and 2) compared to the oral route of administration becomes even more apparent after normalization for differences in dose.

**Table 4: Comparison of the mean pharmacokinetic parameters of the different formulations after dose normalization per mg.**

| Dosing | Cₘₐₓ/mg | AUC₀₋₃₆₀/mg |
|---|---|---|
| | [ng/ml] | [min*ng/ml] |
| Comparison | 33 | 26 |
| wafer A vs. oral gavage | | |
| Comparison | 144 | 121 |
| wafer B vs. oral gavage | | |
| Comparison | 3.62 | 4.75 |
| wafer B vs. wafer A | | |

As shown in Table 4: The comparison on a dose-normalized basis shows that wafer A lead to a 26-fold higher AUC (33-fold Cₘₐₓ) than the oral formulation on a dose-normalized level and wafer B leads to a 121-fold higher AUC (144-fold Cₘₐₓ) in comparison to the oral route. The mono-molecular dispersion wafer B leads to > 4-fold higher exposure of idebenone compared to the suspension type wafer A.

In summary, it can be concluded that
- Oral-mucosal administration of idebenone by a thin film formulation (oral wafer) based on the solid-solution technology strongly improves the relative bioavailability of idebenone by approximately 100-fold over oral administration. The main reason for the increased bioavailability is the initial bypass of the enterohepatic circulation.
- On top of that, there is evidence that the absorption of idebenone from this wafer is increased compared to oral administration of idebenone by gavage.
- A wafer, based on a micronized suspension as shown in Example 1, also improves the relative bioavailability of idebenone, though to a lesser extent.
- The metabolic spectrum of idebenone is comparable after oral administration by gavage and the oral-mucosal application.
   ■ The oral wafer formulation offers the following advantages over the formulations administered via the oral route:
      o Significant dose reduction (less side effects)
      o Higher plasma levels achievable
      o Increased patient convenience
      o Reduced food effect
      o Possibility of administration to patients with swallowing difficulties

## Claims

1. Thin film drug delivery system for the transmucosal administration of a pharmaceutical drug comprising 0.01-80% by weight of an active ingredient of the formula (I) 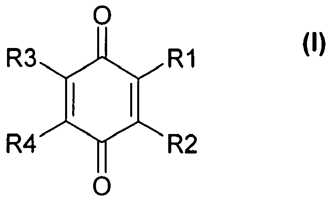 wherein
R1 is a C1-4 alkyl group;
R2 is a hydrogen atom, an optionally substituted alkyl or an optionally substituted alkenyl group;
each of R3 and R4 represents independently an optionally substituted C1-6 alkyl or C1-3 alkoxy group, or
R3 and R4 taken together represent a butadienylene group; and
20-99.99% by weight of a carrier material.

2. The system of claim 1, wherein R1 is a C1-3 alkyl group.

3. The system of claim 1 or 2, wherein R2 is hydrogen, C1-50 alkyl or C2-50 alkenyl, wherein the alkyl or alkenyl may be substituted by 1 to 10 substituents selected from the group consisting of (i) C1-4 alkyl, (ii) hydroxy, (iii) oxo, (iv) amino, (v) mono-C1-6 alkylamino, (vi) di-C1-6 alkylamino, (vii) carboxy, (viii) C1-4 alkoxycarbonyl, (ix) C6-14 aryl, which may be substituted by 1 or 2 substituents selected from the group consisting of C1-4 alkyl, hydroxy, carboxy and C1-6 alkoxycarbonyl, (x) 5- or 6-membered heterocyclic group, which may be substituted by 1 or 2 substituents selected from the group consisting of C1-4 alkyl, hydroxy, carboxy and C1-6 alkoxycarbonyl, and (xi) halogen.

4. The system of any of claims 1 to 3, wherein R2 is C1-22 alkyl or C2-22 alkenyl, wherein the alkyl or alkenyl may be substituted by 1 to 10 substituents selected from the group consisting of (i) C1-4 alkyl, (ii) hydroxy, (iii) oxo, (iv) amino, (v) mono-C1-6 alkylamino, (vi) di-C1-6 alkylamino, (vii) carboxy, (viii) C1-4 alkoxycarbonyl, (ix) C6-14 aryl, which may be substituted by 1 or 2 substituents selected from the group consisting of C1-4 alkyl, hydroxy, carboxy and C1-6 alkoxycarbonyl, (x) 5- or 6-membered heterocyclic group, which may be substituted by 1 or 2 substituents selected from the group consisting of C1-4 alkyl, hydroxy, carboxy and C1-6 alkoxycarbonyl, and (xi) halogen.

5. The system of any of claims 1 to 4, wherein each of R3 and R4 represents independently a C1-6 alkyl, the alkyl being optionally substituted by 1 to 3 substituents selected from the group consisting of hydroxy, halogen, nitro, C1-3 alkyl, C1-3 haloalkyl, carboxy, C1-6 alkoxycarbonyl, 3-pyridyl, 1-imidazolyl and 5-thiazolyl; C1-3 alkoxy, preferably methoxy; or R3 and R4 taken together represent a butadienylene group optionally substituted by 1 to 3 substituents selected from the group consisting of C1-3 alkyl, C1-3 alkoxy, hydroxy, nitro and halogen.

6. The system of any of claims 1 to 5, wherein each of R3 and R4 represents independently C1-4 alkyl, the alkyl being optionally substituted by 1 to 3 substituents selected from the group consisting of hydroxy, halogen, nitro, C1-3 alkyl, C1-3 haloalkyl, carboxy, C1-6 alkoxycarbonyl, 3-pyridyl, 1-imidazolyl and 5-thiazolyl; or C1-3 alkoxy, preferably methoxy.

7. The system of any of claims 1 to 6, wherein said active ingredient is selected from the group consisting of idebenone or an idebenone analogue, preferably idebenone.

8. The system of any of claims 1 to 7, wherein the carrier material is selected from the group consisting of cellulose, a cellulose derivative, polyvinyl alcohol, a poly-N-vinylpyrrolidone, a vinylpyrrolidone-vinyl acetate copolymer, starch, a starch derivative, gelatine, a gelatine derivative and a combination thereof.

9. The system of claim 8, wherein the cellulose derivate is selected from the group consisting of methylcellulose, ethylcellulose, hydroxypropylcellulose, hydroxyethylcellulose, hydroxypropylmethylcellulose (HPMC), carboxymethylcellulose (CMC) and a combination thereof.

10. The system of any of claims 1 to 9, wherein the system contains a suspension of the active ingredient, comprising 30-60% by weight, preferably 40-50% by weight, of the active ingredient, and 40-70% by weight, preferably 50-60% by weight, of a polyvinyl alcohol, a cellulose derivate and/or a starch derivative as carrier material.

11. The system of any of claims 1 to 9, wherein the system contains a solubilised form (mono-molecular dispersion) comprising 3-20% by weight, preferably 5-10% by weight, of the active ingredient and 80-97% by weight, preferably 90-95% by weight, of a water soluble polymer, preferably a substituted carbohydrate, as carrier material.

12. The system of any of claims 1 to 9, wherein the system contains an emulsion comprising 3-50% by weight, preferably 5-30% by weight, of the active ingredient and 50-97% by weight, preferably 70-95% by weight, of a cellulose derivate as carrier material.

13. The system of any of claims 1 to 12 for use in the treatment of a mitochondrial disease, preferably selected from the group consisting of Leber's hereditary optic neuropathy (LHON), autosomal dominant optic atrophy (DOA), macular degeneration, glaucoma, retinopathy, cataract, optic disc drusen (ODD), mitochondrial myopathy, encephalomyopathy, lactic acidosis, stroke-like symptoms (MELAS), myoclonic epilepsy with ragged red fibers (MERRF), myoneurogenic gastrointestinal encephalomyopathy (MNGIE), Kearns-Sayre syndrome, CoQ10 deficiency, and mitochondrial complex deficiencies (1-5, CPEO);
a neurodegenerative disease, preferably selected from the group consisting of Friedreich's ataxia (FRDA), amyotrophic lateral sclerosis (ALS), Parkinson's disease, Alzheimer's disease, Huntington's disease, stroke/reperfusion injury, and dementia;
a neuromuscular disease, preferably selected from the group consisting of Duchenne muscular dystrophy (DMD), Becker muscular dystrophy (BMD), Limb-Girdle muscular dystrophy (LGMD), X-linked dilated cardiomyopathy (XLDCM), Pantothenate kinase-associated neurodegeneration (PKAN), spinal muscular atrophy (SMA), multiple sclerosis and primary progressive multiple sclerosis (PP-MS), Kugelberg-Welander disease, and Werdnig-Hoffmann disease;
a psychiatric disorder, preferably selected from the group consisting of schizophrenia, major depressive disorder, bipolar disorder, and epilepsy;
a metabolic disorder, preferably selected from the group consisting of ageing-related physical decline, obesity, overweight, type II diabetes, and metabolic syndrome;
cancer; multiple sclerosis; primary progressive multiple sclerosis; or
immune dysfunction, preferably selected from the group consisting of arthritis, psoriasis and rheumatoid arthritis.

14. A method for preparing the system according to any of claims 1 to 12, comprising
a) dissolving or dispersing the active ingredient and the carrier in a solvent;
b) drying the solution or dispersion in order to obtain a thin film.
